# EUROPEAN PATENT SPECIFICATION

(11) **EP 3 764 825 B1**
(45) Date of publication and mention of the grant of the patent: **05.10.2022**
(21) Application number: 19708575.6
(22) Date of filing: 11.03.2019
(51) Int. Cl.: A24F 40/85

(54) **CLEANING TOOL FOR HEATING ELEMENT**
REINIGUNGSWERKZEUG FÜR HEIZELEMENT
OUTIL DE NETTOYAGE POUR ÉLÉMENT CHAUFFANT

(30) Priority: 13.03.2018 EP 18161492
(43) Date of publication of application: 20.01.2021
(73) Proprietor: Philip Morris Products S.A., 2000 Neuchâtel (CH)
(72) Inventor: DAYIOGLU, Onur, 2000 Neuchâtel (CH)
(74) Representative: Siepmann, Felix
(86) International application number: PCT/EP2019/056014
(87) International publication number: WO 2019/175099

(56) References cited:
- EP-A1- 2 201 850
- EP-A1- 3 136 887
- EP-A2- 2 797 450
- EP-B1- 2 797 450
- EP-B1- 3 136 887
- GB-A- 608 608
- US-A1- 2017 055 580

## Description

The present invention relates to a cleaning tool for cleaning an aerosol-generating device.

For generating an inhalable aerosol, aerosol-generating devices are known which heat but not burn an aerosol-generating substrate. The substrate typically comprises an aerosol-former and homogenised tobacco material. The substrate may be wrapped with a wrapping paper and provided in the form of a disposable rod such as a heat stick. The known aerosol-generating device comprises a heating chamber, in which the aerosol-generating substrate can be inserted. A heating element such as a heating blade is also arranged in the heating chamber of the aerosol-generating device. During operation of the aerosol-generating device, the aerosol-generating substrate is penetrated by the heating element and subsequently heated to generate an inhalable aerosol. After depletion of the aerosol-generating substrate, the substrate is removed from the heating chamber of the aerosol-generating device. A fresh aerosol-generating substrate can then be inserted into the heating chamber. However, residues of the aerosol-generating substrate may remain in the heating chamber and on the heating element.

US 2017/0055580 A1 relates to an article for use with an apparatus for heating smokable material. The apparatus comprises a heater zone and a heater element that projects into the heater zone. The apparatus may comprise an opening at a first end of the heater zone through which a portion of the article is insertable into the heater zone. The article may comprise a wiper, which comprises an abrasive pad. The wiper may comprise a corrugated member or a member having a plurality of lumps or protrusions extending therefrom.

GB 608,608 A relates to a cleaning device for cleaning the bore of the shank of a smoking pipe.

EP 2 201 850 A1 is directed to a cleaning article configured for cleaning an electrically heated smoking system. The cleaning article may comprise a cleaning brush, a swab, wipe or cloth, or another means for mechanically cleaning the inside of a cavity of the smoking system.

Thus, there is a need for a device for cleaning the heating chamber and the heating element of the aerosol-generating device after operation and removal of the aerosol-generating substrate.

According to a first aspect of the invention there is provided a cleaning tool for cleaning an aerosol-generating device according to claim 1. The aerosol-generating device comprises a heating chamber and a heating element arranged in the heating chamber. The cleaning tool comprises an elongate element that has a proximate end and a distal end. The elongate element comprises an abrasive surface and is configured to be inserted into the heating chamber of the aerosol-generating device for cleaning at least the heating element.

The cleaning tool has a length such that the tool can be inserted into the heating chamber of the aerosol-generating device and during the process held by a user. The abrasive surface of the elongate element is configured for scraping off of residues of the aerosol-generating substrate from the heating element. Preferably, the elongate element is also suited for scraping off of residues of the aerosol-generating substrate from the heating chamber of the aerosol-generating device. The abrasive surface of the elongate element preferably extends along the side surface of the elongate element. In this way, the length of the heating element can be cleaned by sliding the abrasive surface of the elongate element along the length of the heating element. The abrasive surface of the elongate element may also be arranged such that the cleaning tool together with the elongate element and the abrasive surface can be rotated around the heating element so that the abrasive surface comes into contact with the circumference of the heating element.

The elongate element may be made from cardboard, preferably corrugated cardboard, wherein the corrugations preferably extend along the longitudinal length of the elongate element.

Cardboard is a readily available, environmentally friendly and cheap material with relatively high stability. In this way, the cleaning tool can be provided with the mechanical stability to facilitate the cleaning operation within the heating chamber of the aerosol-generating device. At the same time, the cleaning tool can be provided as a cheap disposable so that the tool can be for example added to a pack of heat sticks. The overall dimensions of the cleaning tool are such that the tool fits into a conventional package for heat sticks.

The distal end of the elongate element may be tapered. By providing a tapered end, the corners at the base of the heating chamber of the aerosol-generating device can be reached by the elongate element of the cleaning tool.

The distal end of the elongate element may comprise a rounded portion. The rounded portion facilitates the cleaning of the heating chamber of the aerosol-generating device, particularly the base of the heating chamber. The rounded portion furthermore generates sufficient stability of the distal end of the elongate element such that the distal end of the elongate element is not damaged or deformed during the cleaning operation.

The abrasive surface of the elongate element may be flat. With the flat surface, the contact area between the elongate element and the heating element may be maximized to facilitate cleaning of the heating element of the aerosol-generating device. Particularly, if the heating element is provided as a heating blade, the side surfaces of the heating element can be optimally cleaned with the flat configuration of the abrasive surface of the elongate element.

The abrasive surface of the elongate element may have a curved surface in the direction perpendicular to the longitudinal axis of the elongate element. In other words, the abrasive surface may be provided curved in the radial direction. In this way, the surface may match the surface shape of the heating chamber, particularly if the heating chamber has a cylindrical shape. Thus, this configuration of the abrasive surface of the elongate element enables an optimized cleaning of the heating element as well as of the heating chamber of the aerosol-generating device.

The elongate element may comprise a rib along the longitudinal axis of the elongate element. The rib may be arranged opposite to the abrasive surface of the elongate element. The rib is preferably centrally aligned along the longitudinal axis of the elongate element. The rib may enhance the structural stability of the elongate element so that in use of the cleaning tool the elongate element is more resilient to damage or deformation.

The abrasive surface of the elongate element is configured as a corrugated cardboard surface. This has the advantage that - during insertion of the cleaning tool into the heating chamber of the aerosol-generating device - the heating element may slide in between the corrugations of the corrugated surface so that unwanted residues are scrapped off of the heating element during the insertion and removing of the cleaning tool. Particularly if the elongate element is made from corrugated cardboard, this configuration may enhance the stability of the elongate element and at the same time enable an optimized cleaning operation of the heating element and the heating chamber of the aerosol-generating device. If the heating element is provided as a heating blade, the corrugations may be shaped so that the heating blade can be slided in-between corrugations of the elongate element. Thus, residues may be scraped of the heating blade by sliding the heating blade in the corrugations of the elongate element.

The cleaning tool further comprises a tubular element at the proximal end of the elongate element, which is configured to be insertable into the heating chamber of the aerosol-generating device. In this embodiment, the elongate element may be provided for cleaning of the heating element, while the tubular element may be provided for cleaning of the heating chamber of the aerosol-generating device. During use, the user may clean the heating element and subsequently reverse the cleaning tool for cleaning of the heating chamber with the elongate element or vice versa.

The tubular element comprises at least one groove, preferably a plurality of grooves, for contacting and cleaning the base of the heating chamber. The grooves enhance the cleaning action of the base of the heating chamber. During the cleaning operation, the user may rotate the tubular element after inserting the elongate element into the heating chamber so that the tubular element contacts the base of the heating chamber. The grooves enhance the scraping off of residues of aerosol-forming substrate from the base of the heating chamber.

The tubular element may have a partial or full tubular cross-section. The heating chamber of aerosol-generating devices may have a cylindrical shape. If the shape is of perfectly round cylindrical shape, the shape of the tubular element may be chosen having a full tubular cross section. In this embodiment, the outer shape of the tubular element preferably resembles the shape of the inner surface of the heating chamber. The inner surface of the heating chamber can then be cleaned by inserting the tubular element into the heating chamber. If, however, the inner shape of the heating chamber does not resemble a round cylinder, a partial tubular cross section may be chosen for the shape of the tubular element. For example, a rib or band or protrusion may be provided at the inner surface of the heating chamber. In such a case, the tubular element with the partial tubular cross section may be inserted into the heating chamber only contacting essentially half of the inner surface of the heating chamber opposite from the protrusion. Then, the tubular element may be rotated until the tubular element abuts the protrusion. Hence, the inner surface of the heating chamber can be cleaned according to this embodiment by inserting and rotating the tubular member.

The tubular element may comprise at least one rib arranged along the longitudinal axis of the cleaning tool at the outer surface of the tubular element. Similar to the embodiment in which the tubular element is provided with a partial tubular cross-section, a heating chamber with a protrusion can be cleaned by providing a rib along the longitudinal length of the tubular element. Preferably, the diameter of the tubular element is in this case smaller than the inner diameter of the heating chamber so that the tubular element can be inserted into the heating chamber even if a protrusion is provided in the heating chamber. Only the rib of the tubular element contacts the inner surface of the heating chamber in this embodiment. After insertion of the tubular element, rotation of the tubular element has the effect that the rib of the tubular element scrapes off residues sticking to the inner surface of the heating chamber.

The invention also relates to an aerosol-generating device and cleaning tool for cleaning the aerosol-generating device according to claim 11.

The heating chamber of the aerosol-generating device may have a cylindrical shape. The heating element may be provided as a heating pin or a heating blade and may be centrally aligned in the heating chamber.

The invention also relates to a method for manufacturing a cleaning tool for cleaning an aerosol-generating device according to claim 13.

The invention will be further described, by way of example only, with reference to the accompanying drawings in which:
- Fig. 1: shows different views of a distal portion of a cleaning tool according to the invention;
- Fig. 2: shows the cleaning tool with an additionally attached partial tubular element;
- Fig. 3: shows the cleaning tool with an additionally attached full tubular element;
- Fig. 4: shows the cleaning tool with a tubular element with a rib; and
- Fig. 5: shows the cleaning tool with a tubular element with multiple ribs.

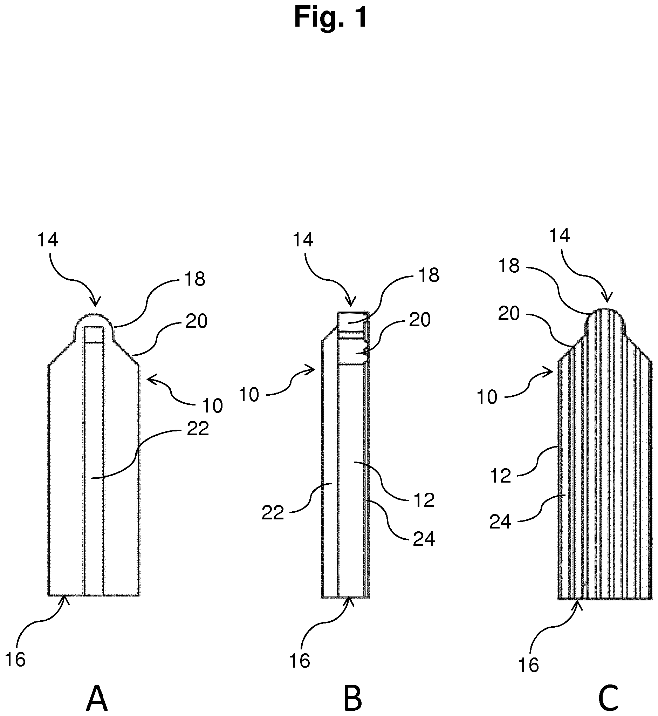

In Figure 1, a distal portion of a cleaning tool 10 is depicted. The distal portion of the cleaning tool 10 is provided for cleaning an aerosol-generating device. Particularly, the distal portion of the cleaning tool is provided for cleaning a heating element and a heating chamber of an aerosol-generating device. The aerosol-generating device may comprise further components such as a housing, a battery for powering the heating element and a controller for controlling the supply of electrical power from the battery to the heating element. The heating chamber may be provided as a cylindrical heating chamber such that a tobacco plug can be inserted into the heating chamber so that the heating element penetrates and heats the tobacco to generate an inhalable aerosol.

Referring again to Figure 1 of the present invention, the distal portion of the cleaning tool 10 comprises an elongate element 12 that has a distal end 14 and a proximate end 16. An abrasive surface is arranged along the elongate element 12.

The distal portion of the cleaning tool 10 is configured to be inserted into a heating chamber of an aerosol-generating device. Particularly, the distal end 14 of the distal portion of the cleaning tool 10 is inserted first into the heating chamber of the aerosol-generating device. Inside of the heating chamber of the aerosol-generating device, a heating element is provided which can be cleaned by the abrasive surface along the elongated element 12 of the distal portion of the cleaning tool 10.

In this regard, the abrasive surface along the elongate element 12 of the distal portion of the cleaning tool 10 is arranged such that the abrasive surface can scrape off residues which adhere to the heating element within the heating chamber of the aerosol-generating device. The tip at the distal end 14 of the distal portion of the cleaning tool 10 may additionally be provided to scrape off residues from the base of the heating chamber. The abrasive surface can also be used to scrape off the residues from the inner walls of the heating chamber by sliding the abrasive surface along the inner walls of the heating chamber.

As can be seen in the left part of Figure 1, Figure 1A, the distal end 14 of the distal portion of the cleaning tool 10 is provided with a rounded portion 18. The rounded portion 18 increases the contact surface between the distal end 14 and the base of the heating chamber.

Furthermore, the elongate element may be tapered adjacent to the rounded portion 18 so that a tapered area 20 is provided. The tapered area 20 facilitates that the distal end 14 of the distal portion of the cleaning tool 10 can reach the base of the heating chamber and particularly the corners of the base of the heating chamber.

In Figure 1A, it can further be seen that a rib 22 is provided at the back side of the elongate element 12. The rib 22 is provided to increase the stiffness of the distal portion of the cleaning tool 10. In this regard, the distal portion of the cleaning tool 10 is preferably made from cardboard.

The right part of Figure 1, Figure 1C, shows the front portion of the distal portion of the cleaning tool 10 which is provided with the abrasive surface 24. The abrasive surface 24 is provided along the side surface of the elongate element 12. The abrasive surface 24 may be provided by any know means such as the material of the elongate element 12 itself or by gluing abrasive material to the surface of the elongate element 12.

As can also be seen in Figure 1C, the surface of the elongate element 12, which is covered by the abrasive surface 24, exhibits corrugations. The corrugations are preferably provided by the distal portion of the cleaning tool 10 being made of a corrugated cardboard. The corrugations may be provided as depicted in Figure 1C so that the cleaning surface is not completely flat. This embodiment may have the advantage that the heating element to be cleaned by the heating tool 10 can slide in between the corrugations so as to optimally scrape off residues from the heating element. Alternatively, the abrasive surface 24 can be provided flat.

Not depicted in Figure 1 is a possible configuration of the surface of the elongate element 12 covered by the abrasive surface 24, in which the surface is curved. This embodiment may be beneficial in cleaning the inner walls of the heating chamber of the aerosol-generating device. In this regard, the curvature of the curved surface of the elongate element 12 may have a curvature which corresponds to the shape of a cylindrical heating chamber of an aerosol-generating device.

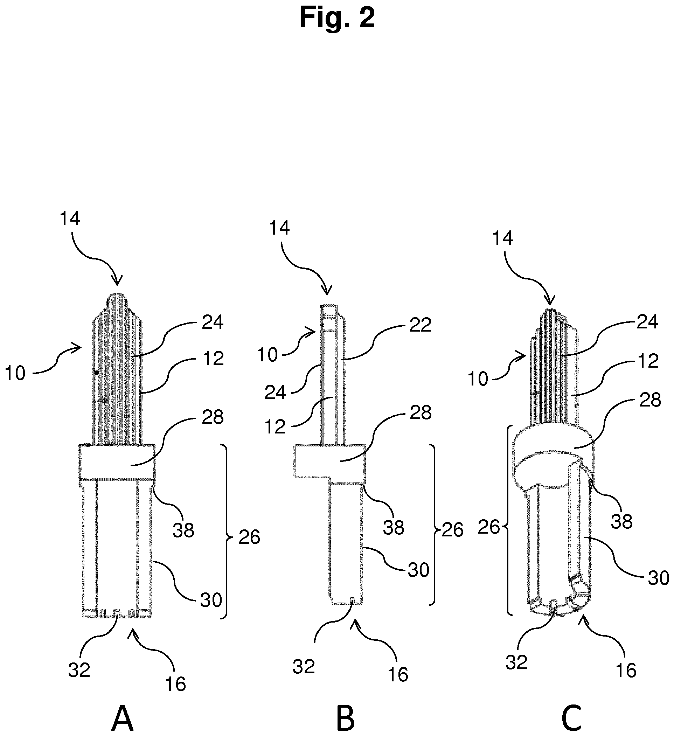

Figure 2 shows a further embodiment of the present invention, in which the cleaning tool 10 is provided with a tubular element 26. The tubular element 26 is provided at the proximate end 16 of the cleaning tool 10. The tubular element 26 is particularly provided to scrape off residues from the base and the inner walls of the heating chamber. In this embodiment, the distal portion of the cleaning tool 10, which is depicted in Figure 1, is provided only for cleaning the heating element in the heating chamber, while the tubular element 26 is provided for cleaning the base and the inner walls of the heating chamber.

The tubular element 26, which is depicted in Figure 2, is connected with the distal portion of the cleaning tool 10 by means of a connection element 28 such as a flange. The connection element 28 may have an outer diameter which is larger than the inner diameter of the heating chamber of the aerosol-generating device. In this way, the connection element 28 may limit the insertion of the distal portion and the tubular element 26 into the heating chamber. The connection element 28 may comprise a handle to facilitate holding of the cleaning tool 10 by a user.

Proximal from the connection element 28, a partially tubular portion 30 of the tubular element is depicted in Figure 2. The partial tubular portion 30 may have a shape that resembles the inner walls of a heating chamber of an aerosol-generating device. At the proximal end 16 of the partial tubular portion 30, one or more grooves 32 can be provided. During insertion of the partial tubular portion 30 into the heating chamber of an aerosol-generating device, the partial tubular portion 30 is inserted until the grooves 32 contact the base of the heating chamber. Then, the cleaning tool 10 is rotated such that residues are scraped off the base of the heating chamber by means of the grooves 32. Furthermore, during rotation, residues can be scraped off the inner walls of the heating chamber by the outer surface of the partial tubular portion 30. The outer surface of the partial tubular portion 30 can be provided with an abrasive surface to facilitate removal of unwanted residues.

The configuration of the tubular portion 26 as a partial tubular portion 30 has the advantage that this portion can be inserted into the heating chamber of an aerosol-generating device even if the heating chamber does not have a perfectly cylindrical shape. For example, a protrusion such as a vertical ridge may be presented in the heating chamber such that a full tubular portion could not be inserted into the heating chamber or that a full tubular portion would then not contact the inner walls of the heating chamber so that residues could not be sufficiently removed from the inner walls.

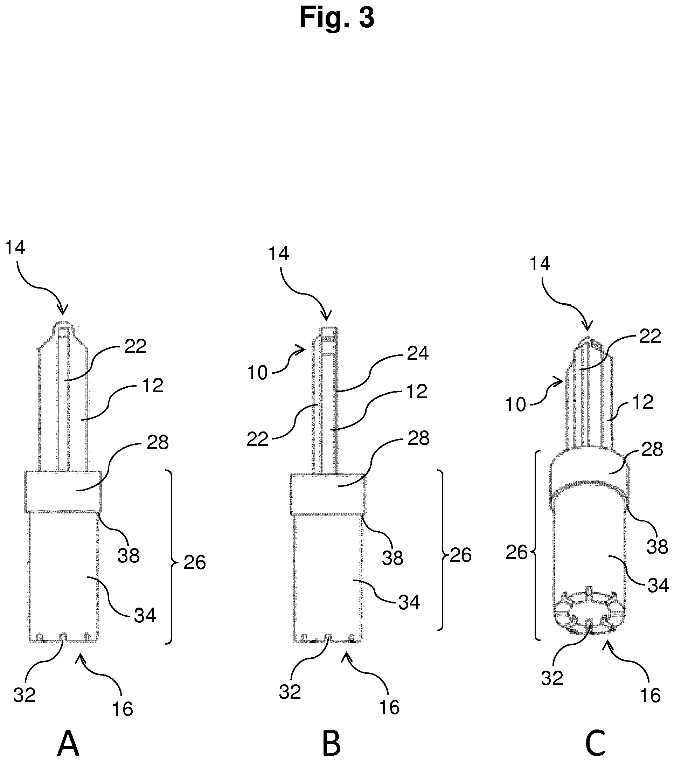

If, however, the heating chamber has a perfectly cylindrical shape, a full tubular portion 34 as depicted in Figure 3 could be employed, wherein a rotation of this full tubular portion 34 can optimally remove unwanted residues from the inner walls of the heating chamber of the aerosol-generating device. The tubular portion 26, more preferably the full tubular portion 34, is hollow so that the tubular portion 26 can be inserted into the heating chamber and not be obstructed by the heating element.

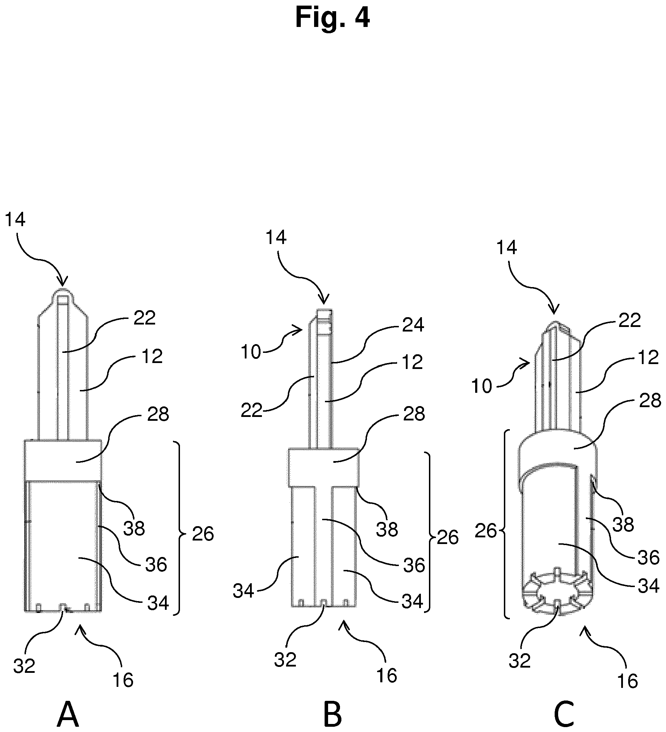

Figure 4 shows a configuration, in which a rib 36 is arranged along the length of the tubular portion 26. In the embodiment depicted in Figure 4, the tubular portion 26 is provided as a full tubular portion 34. In this embodiment, the rib 36 predominantly scrapes off residues from the inner walls of the heating chamber by rotation of the cleaning tool 10. The rib 36 is configured to come into contact with the inner walls of the heating chamber during insertion of the tubular portion 26 into the heating chamber.

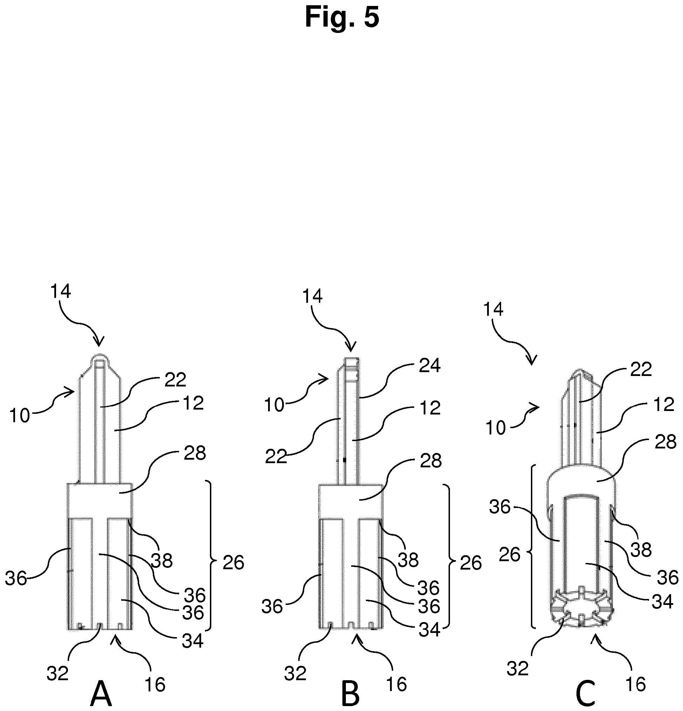

In Figure 5, multiple ribs 36 are depicted. Multiple ribs 36 can be provided to increase the cleaning action of the cleaning tool during rotation of the cleaning tool. On the other hand, the number of ribs 36 can be adapted to the shape of the heating chamber of the aerosol-generating device. For example, if multiple protrusions or other structures are presented in the heating chamber, corresponding structures such as the depicted ribs 36 can be provided at the tubular element 26 such that a cleaning action of the base as well as the inner walls of the heating chamber can be facilitated.

During use, the tubular element 26 is inserted into the heating chamber of the aerosol-generating device to clean the heating chamber, particularly the inner walls and the base of the heating chamber. In this first cleaning step, the distal portion may act as a handle. Subsequently, the cleaning tool 10 is reversed so that the distal portion depicted in Figure 1 is inserted into the heating chamber for cleaning of the heating element. In this second cleaning step, the tubular element 26 may act as a handle. The rounded portion 18 may be used to remove the residues from the base of the heating chamber after them being loosened by the tubular element 26. The cleaning tool 10 may also be used vice versa for cleaning of the aerosol-generating device.

As depicted in Figures 2 to 5, a shoulder 38 can be provided between the connection element 28 and the tubular portion 30, 34 so that the cleaning tool 10 can be inserted into the heating chamber until the connection element 28 and/or shoulder 38 abuts the heating chamber. Preferably, the shoulder 38 is provided such that the grooves 32 contact the base of the heating chamber when the shoulder 38 contacts the aerosol-generating device thereby limiting the further insertion of the cleaning tool 10 into the heating chamber.

## Claims

1. Cleaning tool (10) for cleaning an aerosol-generating device, wherein the aerosol-generating device comprises a heating chamber and a heating element arranged in the heating chamber, wherein the cleaning tool (10) comprises an elongate element (12) that has a proximate end (16) and a distal end (14), wherein the elongate element (12) comprises an abrasive surface (24) and is configured to be inserted into the heating chamber of the aerosol-generating device for cleaning at least the heating element, wherein the abrasive surface (24) of the elongate element (12) is configured as a corrugated cardboard surface, wherein the cleaning tool (10) further comprises a tubular element (26) at the proximal end (16) of the elongate element (12), the tubular element (26) being configured to be inserted into the heating chamber of the aerosol-generating device, and wherein the tubular element (26) comprises at least one groove (32) for contacting and cleaning the base of the heating chamber.

2. A cleaning tool (10) according to claim 1, wherein the elongate element (12) is made from cardboard, preferably corrugated cardboard, wherein the corrugations preferably extend along the longitudinal length of the elongate element (12).

3. A cleaning tool (10) according to one of the previous claims, wherein the distal end (14) of the elongate element (12) is tapered.

4. A cleaning tool (10) according to one of the previous claims, wherein the distal end (14) of the elongate element (12) comprises a rounded portion (18).

5. A cleaning tool (10) according to one of the previous claims, wherein the abrasive surface (24) of the elongate element (12) is flat, and is preferably arranged along the side surface of the elongate element (12).

6. A cleaning tool (10) according to one claims 1 to 4, wherein the abrasive surface (24) of the elongate element (12) has a curved surface in the direction perpendicular to the longitudinal axis of the elongate element (12), and is preferably arranged along the side surface of the elongate element (12).

7. A cleaning tool (10) according to one of the previous claims, wherein the elongate element (12) comprises a rib (22) along the longitudinal axis of the elongate element (12), and wherein the rib (22) is arranged opposite to the abrasive surface (24) of the elongate element (12), wherein the rib (22) is preferably centrally aligned along the longitudinal axis of the elongate element (12).

8. A cleaning tool (10) according to any of the preceding claims, wherein the tubular element (26) comprises a plurality of grooves (32) for contacting and cleaning the base of the heating chamber.

9. A cleaning tool (10) according to any of the preceding claims, wherein the tubular element (26) has a partial or full tubular cross-section.

10. A cleaning tool (10) according to any of the preceding claims, wherein the tubular element (26) comprises at least one rib (36) arranged along the longitudinal axis of the cleaning tool (10) at the outer surface of the tubular element (26).

11. Aerosol-generating device and cleaning tool (10) according to any of claims 1 to 10 for cleaning the aerosol-generating device, wherein the aerosol-generating device comprises a heating chamber and a heating element arranged in the heating chamber, wherein the cleaning tool (10) comprises an elongate element (12) that has a proximate end (16) and a distal end (14), wherein the elongate element (12) comprises an abrasive surface (24) and is configured to be inserted into the heating chamber of the aerosol-generating device for cleaning at least the heating element, wherein the abrasive surface (24) of the elongate element (12) is configured as a corrugated cardboard surface.

12. Aerosol-generating device and cleaning tool (10) according to claim 11, wherein the heating chamber of the aerosol-generating device has a cylindrical shape, and wherein the heating element is provided as a heating pin or a heating blade and is centrally aligned in the heating chamber.

13. A method for manufacturing a cleaning tool (10) for cleaning an aerosol-generating device, wherein the aerosol-generating device comprises a heating chamber and a heating element arranged in the heating chamber, the method comprising the steps of:
(a) providing the cleaning tool (10) with a proximate end (16) and a distal end (14) and an elongate element (12) at the distal end (14),
(b) providing the elongate element (12) with an abrasive surface (24), wherein the abrasive surface (24) of the elongate element (12) is configured as a corrugated cardboard surface,
(c) configuring the elongate element (12) insertable into the heating chamber of the aerosol-generating device for cleaning at least the heating element,
(d) providing the cleaning tool (10) with a tubular element (26) at the proximal end (16) of the elongate element (12), the tubular element (26) being configured to be inserted into the heating chamber of the aerosol-generating device, and
(e) providing the tubular element (26) with at least one groove (32) for contacting and cleaning the base of the heating chamber.

## Patentansprüche

1. Reinigungswerkzeug (10) zum Reinigen einer Aerosolerzeugungsvorrichtung, wobei die Aerosolerzeugungsvorrichtung eine Heizkammer und ein in der Heizkammer angeordnetes Heizelement umfasst, wobei das Reinigungswerkzeug (10) ein längliches Element (12) umfasst, das ein proximales Ende (16) und ein distales Ende (14) aufweist, wobei das längliche Element (12) eine abrasive Fläche (24) aufweist und zum Einführen in die Heizkammer der Aerosolerzeugungsvorrichtung zum Reinigen zumindest des Heizelements ausgebildet ist, wobei die abrasive Fläche (24) des länglichen Elements (12) als eine Wellpappenfläche ausgebildet ist, wobei das Reinigungswerkzeug (10) ferner ein rohrförmiges Element (26) am proximalen Ende (16) des länglichen Elements (12) aufweist, wobei das rohrförmige Element (26) zum Einführen in die Heizkammer der Aerosolerzeugungsvorrichtung ausgebildet ist, und wobei das rohrförmige Element (26) wenigstens eine Nut (32) zum Kontaktieren und Reinigen des Bodens der Heizkammer aufweist.

2. Reinigungswerkzeug (10) nach Anspruch 1, wobei das längliche Element (12) aus Pappe, bevorzugt aus Wellpappe, hergestellt ist, wobei sich die Wellungen bevorzugt entlang der Längslänge des länglichen Elements (12) erstrecken.

3. Reinigungswerkzeug (10) nach einem der vorhergehenden Ansprüche, wobei das distale Ende (14) des länglichen Elements (12) verjüngt ist.

4. Reinigungswerkzeug (10) nach einem der vorhergehenden Ansprüche, wobei das distale Ende (14) des länglichen Elements (12) einen abgerundeten Abschnitt (18) aufweist.

5. Reinigungswerkzeug (10) nach einem der vorhergehenden Ansprüche, wobei die abrasive Fläche (24) des länglichen Elements (12) flach ist und bevorzugt entlang der Seitenfläche des länglichen Elements (12) angeordnet ist.

6. Reinigungswerkzeug (10) nach einem der Ansprüche 1 bis 4, wobei die abrasive Fläche (24) des länglichen Elements (12) eine gekrümmte Fläche in der Richtung senkrecht zur Längsachse des länglichen Elements (12) aufweist und bevorzugt entlang der Seitenfläche des länglichen Elements (12) angeordnet ist.

7. Reinigungswerkzeug (10) nach einem der vorhergehenden Ansprüche, wobei das längliche Element (12) eine Rippe (22) entlang der Längsachse des länglichen Elements (12) aufweist, und wobei die Rippe (22) gegenüber der abrasiven Fläche (24) des länglichen Elements (12) angeordnet ist, wobei die Rippe (22) bevorzugt mittig entlang der Längsachse des länglichen Elements (12) ausgerichtet ist.

8. Reinigungswerkzeug (10) nach einem beliebigen der vorhergehenden Ansprüche, wobei das rohrförmige Element (26) eine Vielzahl von Nuten (32) zum Kontaktieren und Reinigen des Bodens der Heizkammer aufweist.

9. Reinigungswerkzeug (10) nach einem beliebigen der vorhergehenden Ansprüche, wobei das rohrförmige Element (26) einen teilweisen oder vollständigen rohrförmigen Querschnitt aufweist.

10. Reinigungswerkzeug (10) nach einem beliebigen der vorhergehenden Ansprüche, wobei das rohrförmige Element (26) wenigstens eine Rippe (36) aufweist, die entlang der Längsachse des Reinigungswerkzeugs (10) an der Außenfläche des rohrförmigen Elements (26) angeordnet ist.

11. Aerosolerzeugungsvorrichtung und Reinigungswerkzeug (10) nach einem der Ansprüche 1 bis 10 zur Reinigung der Aerosolerzeugungsvorrichtung, wobei die Aerosolerzeugungsvorrichtung eine Heizkammer und ein in der Heizkammer angeordnetes Heizelement aufweist, wobei das Reinigungswerkzeug (10) ein längliches Element (12) umfasst, das ein proximales Ende (16) und ein distales Ende (14) aufweist, wobei das längliche Element (12) eine abrasive Fläche (24) aufweist und zum Einführen in die Heizkammer der Aerosolerzeugungsvorrichtung zum Reinigen wenigstens des Heizelements ausgelegt ist, wobei die abrasive Fläche (24) des länglichen Elements (12) als eine Wellpappfläche ausgelegt ist.

12. Aerosolerzeugungsvorrichtung und Reinigungswerkzeug (10) nach Anspruch 11, wobei die Heizkammer der Aerosolerzeugungsvorrichtung eine zylindrische Form aufweist, und wobei das Heizelement als Heizstift oder Heizblatt vorgesehen und mittig in der Heizkammer ausgerichtet ist.

13. Verfahren zur Herstellung eines Reinigungswerkzeugs (10) zur Reinigung einer Aerosolerzeugungsvorrichtung, wobei die Aerosolerzeugungsvorrichtung eine Heizkammer und ein in der Heizkammer angeordnetes Heizelement aufweist, wobei das Verfahren die folgenden Schritte umfasst:
(a) Vorsehen des Reinigungswerkzeugs (10) mit einem proximalen Ende (16) und einem distalen Ende (14) und einem länglichen Element (12) an dem distalen Ende (14),
(b) Vorsehen des länglichen Elements (12) mit einer abrasiven Fläche (24), wobei die abrasive Fläche (24) des länglichen Elements (12) als eine Wellpappenfläche ausgelegt ist,
(c) Auslegen des in die Heizkammer der Aerosolerzeugungsvorrichtung einführbaren länglichen Elements (12) zur Reinigung wenigstens des Heizelements,
(d) Vorsehen des Reinigungswerkzeugs (10) mit einem rohrförmigen Element (26) am proximalen Ende (16) des länglichen Elements (12), wobei das rohrförmige Element (26) zum Einführen in die Heizkammer der Aerosolerzeugungsvorrichtung ausgelegt ist, und
(e) Vorsehen des rohrförmigen Elements (26) mit wenigstens einer Nut (32) zum Kontaktieren und Reinigen des Bodens der Heizkammer.

## Revendications

1. Outil de nettoyage (10) destiné à nettoyer un dispositif de génération d'aérosol, dans lequel le dispositif de génération d'aérosol comprend une chambre de chauffage et un élément de chauffage disposé dans la chambre de chauffage, dans lequel l'outil de nettoyage (10) comprend un élément allongé (12) qui a une extrémité proximale (16) et une extrémité distale (14), dans lequel l'élément allongé (12) comprend une surface abrasive (24) et est configuré pour être inséré dans la chambre de chauffage du dispositif de génération d'aérosol pour nettoyer au moins l'élément de chauffage, dans lequel la surface abrasive (24) de l'élément allongé (12) est configurée comme une surface de carton ondulé, dans lequel l'outil de nettoyage (10) comprend en outre un élément tubulaire (26) au niveau de l'extrémité proximale (16) de l'élément allongé (12), l'élément tubulaire (26) étant configuré pour être inséré dans la chambre de chauffage du dispositif de génération d'aérosol, et dans lequel l'élément tubulaire (26) comprend au moins une rainure (32) pour venir en contact avec la base de la chambre de chauffage et la nettoyer.

2. Outil de nettoyage (10) selon la revendication 1, dans lequel l'élément allongé (12) est composé de carton, de préférence de carton ondulé, dans lequel les ondulations s'étendent de préférence le long de la longueur longitudinale de l'élément allongé (12).

3. Outil de nettoyage (10) selon l'une des revendications précédentes, dans lequel l'extrémité distale (14) de l'élément allongé (12) est effilée.

4. Outil de nettoyage (10) selon l'une des revendications précédentes, dans lequel l'extrémité distale (14) de l'élément allongé (12) comprend une partie arrondie (18).

5. Outil de nettoyage (10) selon l'une quelconque des revendications précédentes, dans lequel la surface abrasive (24) de l'élément allongé (12) est plate, et est de préférence disposée le long de la surface latérale de l'élément allongé (12).

6. Outil de nettoyage (10) selon l'une quelconque des revendications 1 à 4, dans lequel la surface abrasive (24) de l'élément allongé (12) a une surface incurvée dans la direction perpendiculaire à l'axe longitudinal de l'élément allongé (12), et est de préférence disposée le long de la surface latérale de l'élément allongé (12).

7. Outil de nettoyage (10) selon l'une des revendications précédentes, dans lequel l'élément allongé (12) comprend une nervure (22) le long de l'axe longitudinal de l'élément allongé (12), et dans lequel la nervure (22) est disposée à l'opposé de la surface abrasive (24) de l'élément allongé (12), dans lequel la nervure (22) est de préférence alignée au centre le long de l'axe longitudinal de l'élément allongé (12).

8. Outil de nettoyage (10) selon l'une quelconque des revendications précédentes, dans lequel l'élément tubulaire (26) comprend une pluralité de rainures (32) pour venir en contact avec la base de la chambre de chauffage et la nettoyer.

9. Outil de nettoyage (10) selon l'une quelconque des revendications précédentes, dans lequel l'élément tubulaire (26) a une coupe transversale tubulaire partielle ou complète.

10. Outil de nettoyage (10) selon l'une quelconque des revendications précédentes, dans lequel l'élément tubulaire (26) comprend au moins une nervure (36) disposée le long de l'axe longitudinal de l'outil de nettoyage (10) au niveau de la surface extérieure de l'élément tubulaire (26).

11. Dispositif de génération d'aérosol et outil de nettoyage (10) selon l'une quelconque des revendications 1 à 10 destiné à nettoyer le dispositif de génération d'aérosol. dans lesquels le dispositif de génération d'aérosol comprend une chambre de chauffage et un élément de chauffage disposé dans la chambre de chauffage, dans lequel l'outil de nettoyage (10) comprend un élément allongé (12) qui a une extrémité proximale (16) et une extrémité distale (14), dans lequel l'élément allongé (12) comprend une surface abrasive (24) et est configuré pour être inséré dans la chambre de chauffage du dispositif de génération d'aérosol pour nettoyer au moins l'élément de chauffage, dans lequel la surface abrasive (24) de l'élément allongé (12) est configurée comme une surface de carton ondulé.

12. Dispositif de génération d'aérosol et outil de nettoyage (10) selon la revendication 11, dans lesquels la chambre de chauffage du dispositif de génération d'aérosol a une forme cylindrique, et dans lesquels l'élément de chauffage est prévu sous la forme d'une broche chauffante ou d'une lame chauffante et est aligné au centre dans la chambre de chauffage.

13. Procédé de fabrication d'un outil de nettoyage (10) destiné à nettoyer un dispositif de génération d'aérosol, dans lequel le dispositif de génération d'aérosol comprend une chambre de chauffage et un élément de chauffage disposé dans la chambre de chauffage, le procédé comprenant les étapes de :
(a) fourniture de l'outil de nettoyage (10) avec une extrémité proximale (16) et une extrémité distale (14) et un élément allongé (12) au niveau de l'extrémité distale (14),
(b) fourniture de l'élément allongé (12) avec une surface abrasive (24), dans lequel la surface abrasive (24) de l'élément allongé (12) est configurée comme une surface de carton ondulé,
(c) configuration de l'élément allongé (12) pouvant être inséré dans la chambre de chauffage du dispositif de génération d'aérosol afin de nettoyer au moins l'élément de chauffage,
(d) fourniture de l'outil de nettoyage (10) avec un élément tubulaire (26) au niveau de l'extrémité proximale (16) de l'élément allongé (12), l'élément tubulaire (26) étant configuré pour être inséré dans la chambre de chauffage du dispositif de génération d'aérosol, et
(e) fourniture de l'élément tubulaire (26) avec au moins une rainure (32) pour venir en contact avec la base de la chambre de chauffage et la nettoyer.
